# EUROPEAN PATENT APPLICATION

(11) **EP 3 269 709 A1**
(43) Date of publication of application: **17.01.2018**
(21) Application number: 16761974.1
(22) Date of filing: 09.03.2016
(51) Int. Cl.: C07C 311/49, C07C 311/28, A61K 31/18

(54) **N-PHENYL-N'-PHENOXYCARBONYL-PHENYLSULFONHYDRAZIDE DERIVATIVE AND PHARMACEUTICAL COMPOSITION COMPRISING SAME**

(30) Priority: 10.03.2015 KR 20150033281
(71) Applicant: UNIVERSITY-INDUSTRY COOPERATION GROUP OF KYUNG HEE UNIVERSITY, Giheung-gu, Yongin-si, Gyeonggi-do 17104 (KR)
(72) Inventor: LEE, Jae Yeol, Seoul 03623 (KR); PARK, Eun Beul, Daejeon 35356 (KR); LEE, Sunhoe, Cheongju-si Chungcheongbuk-do 28185 (KR); LEE, Kyung Tae, Seoul 01735 (KR); KIM, Kwangjong, Seoul 02126 (KR); KIM, Minju, Suwon-si Gyeonggi-do 16339 (KR)
(74) Representative: V.O.
(86) International application number: PCT/KR2016/002322
(87) International publication number: WO 2016/144087

(57) **Abstract**

The present invention relates to a *N*-phenyl-*N'*-phenoxycarbonyl-phenylsulfonhydrazide derivative with excellent inhibitory activity on PGE₂ production, a method for preparing the same and a pharmaceutical composition comprising the same as an active ingredient. The present *N*-phenyl-*N'*-phenoxycarbonyl-phenylsulfonhydrazide derivative may be used effectively for preventing or treating inflammation, arthritis, high fever, pain, cancer, stroke or bone disease.

## Description

### [Technical Field]

The present invention is related to N-phenyl-N'-phenoxycarbonyl-phenylsulfonhydrazide derivatives, a method for preparing the same and a pharmaceutical composition comprising the same. More particularly, the present invention is related to N-phenyl-N'-phenoxycarbonyl-phenylsulfonhydrazide derivatives inhibiting PGE₂ production, a method for preparing the same and a pharmaceutical composition comprising the same as an active ingredient.

### [Background Art]

Prostanoid is an endocrine agent functioning in diverse physiological systems. Prostaglandin E₂ (PGE₂) among them is known to be involved in inflammation.

COX-2 inhibitors which inhibit selectively COX-2 enzyme have been developed as a therapeutic agent for inflammation. Representative COX-2 inhibitors are celecoxib (Celebrex™, Pfizer), valdecoxib (Bextra™, G. D. Searle & Company) and rofecoxib (Vioxx™, Merck). They have been widely used for treating arthritis, severe pain, rheumatism, etc.

However, although COX-2 inhibitors have reduced gastrointestinal disorder which is a major side effect of non-steroidal anti-inflammatory drugs (NSAID), many of them, except for celecoxib, were taken off the market due to cardiovascular side effects. It has been reported that such cardiovascular side effects are caused because PGI₂ (prostacyclin) and TXA₂ (thromboxane) production, as well as PGE₂ production, are inhibited due to inhibition of PGH₂ (prostaglandin H₂) production.

Accordingly, an inhibitor which inhibits selectively microsomal prostaglandin E₂ synthase-1 (mPGES-1) which is involved in PGE₂ production by acting in terminal step of PGH₂ is considered as a new drug candidate addressing disadvantages of COX-2 inhibitors. Researches targeting mPGES-1 are under way.

In particular, a pain and inflammation animal model study has shown that mPGES-1 inhibition is as effective as treatment with non-steroidal anti-inflammatory drugs. Thus, mPGES-1 inhibition is expected to be effective in treating inflammation, arthritis, high fever, pain, cancer, stroke, bone disease, etc.

Bioorg. Med. Chem. Lett. 22 (2012) 7335-7339 reports a compound of the following formula (1) as a new mPGES-1 inhibitor candidate. However, the inhibitory effect of the compound on PGE₂ production is not sufficient for development as a pharmaceutical product.

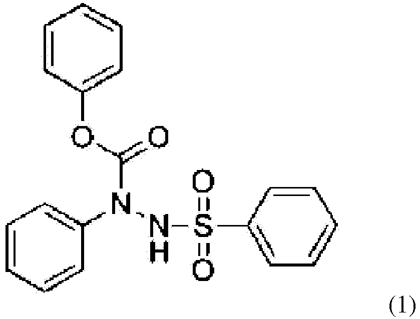

### [Disclosure]

### [Technical Problem]

An object of the present invention is to provide a N-phenyl-N'-phenoxycarbonyl-phenylsulfonhydrazide derivative of the following formula (I) or pharmaceutically acceptable salt thereof which has excellent inhibitory activity on PGE₂ production.

Another object of the present invention is to provide a method for preparing a *N*-phenyl-*N'*-phenoxycarbonyl-phenylsulfonhydrazide derivative of the following formula (I) or pharmaceutically acceptable salt thereof.

Still another object of the present invention is to provide a pharmaceutical composition for inhibiting mPGES-1 comprising a *N*-phenyl-*N*'-phenoxycarbonyl-phenylsulfonhydrazide derivative of the following formula (I) or pharmaceutically acceptable salt thereof.

### [Technical Solution]

One aspect of the present invention relates to a *N*-phenyl-*N'*-phenoxycarbonyl-phenylsulfonhydrazide derivative of the following formula (I) or pharmaceutically acceptable salt thereof. 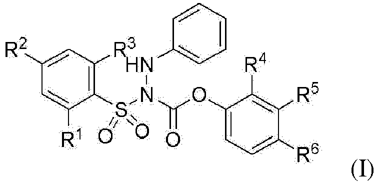 wherein,
R¹ is hydrogen, C₁-C₆ alkyl or C₁-C₆ alkoxy,
R² is C₁-C₆ alkyl, C₁-C₆ alkoxy, halogen, C₁-C₆ haloalkyl or aryl,
R³ is hydrogen, C₁-C₆ alkyl or C₁-C₆ alkoxy,
R⁴ is hydrogen, C₁-C₆ alkyl or C₁-C₆ alkoxy,
R⁵ is hydrogen, C₁-C₆ alkyl or C₁-C₆ alkoxy, and
R⁶ is hydrogen, C₁-C₆ alkyl, C₁-C₆ alkoxy or aryloxy, or
R⁵ and R⁶ are taken together with the carbon atom to which they are attached to form a 4 to 7-membered hydrocarbon ring.

The term "C₁-C₆ alkyl" as used herein means a straight or branched hydrocarbon having 1 to 6 carbon atoms, which includes methyl, ethyl, n-propyl, i-propyl, n-butyl, i-butyl, t-butyl, etc., but is not limited thereto.

The term "C₁-C₆ alkoxy" as used herein means a straight or branched alkoxy having 1 to 6 carbon atoms, which includes methoxy, ethoxy, n-propanoxy, etc., but is not limited thereto.

The term "C₁-C₆ haloalkyl" as used herein means a straight or branched hydrocarbon having 1 to 6 carbon atoms substituted with at least one halogen selected from the group consisting of fluorine, chlorine, bromine and iodine, which includes trifluoromethyl, trichloromethyl, etc., but is not limited thereto.

The term "aryl" as used herein includes all of aromatic group, heteroaromatic group and partially reduced derivatives thereof. The aromatic group means a 5 to 15-membered simple or fused ring. The heteroaromatic group means an aromatic group containing at least one atom selected from oxygen, sulfur and nitrogen. Examples of the aryl include phenyl, naphthyl, pyridinyl, furanyl, thiophenyl, indolyl, quinolinyl, imidazolinyl, oxazolyl, thiazolyl, tetrahydronaphthyl, etc., but are not limited thereto.

The term "aryloxy" as used herein means an aryl group singular bonded to oxygen, which includes phenoxy, benzyloxy, etc., but is not limited thereto.

One or more hydrogen of the C₁-C₆ alkyl, C₁-C₆ alkoxy, C₁-C₆ haloalkyl, aryl and aryloxy may be substituted with C₁-C₆ alkyl, C₂-C₆ alkenyl, C₂-C₆ alkynyl, C₃-C₁₀ cycloalkyl, C₁-C₆ haloalkyl, C₁-C₆ alkoxy, C₁-C₆ thioalkoxy, aryl, acyl, hydroxyl, thio, halogen, amino, alkoxycarbonyl, carboxy, carbamoyl, cyano, nitro, etc.

In one embodiment of the present invention, the *N*-phenyl-*N'*-phenoxycarbonyl-phenylsulfonhydrazide derivative has the above formula (I)
wherein,
R¹ is hydrogen or C₁-C₆ alkyl,
R² is C₁-C₆ alkyl, C₁-C₆ alkoxy, halogen, C₁-C₆ haloalkyl or aryl,
R³ is hydrogen or C₁-C₆ alkyl,
R⁴ is hydrogen or C₁-C₆ alkoxy,
R⁵ is hydrogen, and
R⁶ is hydrogen, C₁-C₆ alkyl, C₁-C₆ alkoxy or aryloxy, or
R⁵ and R⁶ are taken together with the carbon atom to which they are attached to form a 4 to 7-membered hydrocarbon ring.

In one embodiment of the present invention, the N-phenyl-N'-phenoxycarbonyl-phenylsulfonhydrazide derivative has the above formula (I)
wherein,
R¹ is hydrogen or methyl,
R² is methyl, n-propyl, i-propyl, t-butyl, methoxy, chloro, trifluoromethyl or phenyl,
R³ is hydrogen or methyl,
R⁴ is hydrogen or methoxy,
R⁵ is hydrogen, and
R⁶ is hydrogen, methyl, ethyl, methoxy, ethoxy, phenoxy or benzyloxy, or
R⁵ and R⁶ are taken together with the carbon atom to which they are attached to form a 5-membered hydrocarbon ring.

The pharmaceutically acceptable salts of the present invention include nontoxic inorganic acid salts and organic acid salts, including hydrochloride, sulfate, nitrate, phosphate, acetate, adipate, aspartate, benzoate, benzensulfonate, citrate, camphorate, camphorsulfonate, diphosphate, ethanesulfonate, fumarate, glutamate, malate, lactate, methanesulfonate, succinate, tartrate, picrate, tosylate, etc.

The representative compounds according to the present invention are selected from the following group. *N*-phenyl-*N*'-(4-methoxyphenoxycarbonyl)-4-methoxyphenylsulfonhydrazide (I-1); *N*-phenyl-*N*'-(4-methylphenoxycarbonyl)-4-methylphenylsulfonhydrazide (I-2); *N*-phenyl-*N*'-(2-methoxyphenoxycarbonyl)-4-methylphenylsulfonhydrazide (I-3); *N*-phenyl-*N*'-(4-benzyloxyphenoxycarbonyl)-4-methoxyphenylsulfonhydrazide (I-4); *N*-phenyl-*N*'-(4-ethoxyphenoxycarbonyl)-4-methoxyphenylsulfonhydrazide (I-5); *N*-phenyl-*N*'-(4-phenoxyphenoxycarbonyl)-4-methoxyphenylsulfonhydrazide (I-6); *N*-phenyl-*N*'-(4-ethylphenoxycarbonyl)-4-methoxyphenylsulfonhydrazide (I-7); *N*-phenyl-*N*'-(4-benzyloxyphenoxycarbonyl)-4-methylphenylsulfonhydrazide (I-8); *N*-phenyl-*N*'-(2,3-dihydro-1*H*-inden-5-oxycarbonyl)-4-methoxyphenylsulfonhydrazide (I-9); *N*-phenyl-*N*'-phenoxycarbonyl-4-chlorophenylsulfonhydrazide (I-10); *N*-phenyl-*N*'-(2-methoxyphenoxycarbonyl)-4-chlorophenylsulfonhydrazide (I-11); *N*-phenyl-*N*'-(4-ethylphenoxycarbonyl)-4-chlorophenylsulfonhydrazide (I-12); *N*-phenyl-*N*'-(4-ethoxyphenoxycarbonyl)-4-chlorophenylsulfonhydrazide (I-13); *N*-phenyl-*N*'-(4-phenoxyphenoxycarbonyl)-4-chlorophenylsulfonhydrazide (I-14); *N*-phenyl-*N*'-(4-benzyloxyphenoxycarbonyl)-4-chlorophenylsulfonhydrazide (I-15); *N*-phenyl-*N*'-(4-phenoxyphenoxycarbonyl)-2-mesitylenesulfonhydrazide (I-16); *N*-phenyl-*N*'-(4-benzyloxyphenoxycarbonyl)-2-mesitylenesulfonhydrazide (I-17); *N*-phenyl-*N*'-(4-phenoxyphenoxycarbonyl)-biphenyl-4-sulfonhydrazide (I-18); *N*-phenyl-*N*'-(4-benzyloxyphenoxycarbonyl)-biphenyl-4-sulfonhydrazide (I-19); *N*-phenyl-*N*'-(4-phenoxyphenoxycarbonyl)-4-*n*-propylphenylsulfonhydrazide (I-20); *N*-phenyl-*N*'-(4-benzyloxyphenoxycarbonyl)-4-trifluoromethylphenylsulfonhydrazide (I-21); *N*-phenyl-*N*'-(4-benzyloxyphenoxycarbonyl)-4-*t*-butylphenylsulfonhydrazide (I-22); *N*-phenyl-*N*'-(4-phenoxyphenoxycarbonyl)-4-*t*-butylphenylsulfonhydrazide (I-23); *N*-phenyl-*N*'-(4-phenoxyphenoxycarbonyl)-4-*i*-propylphenylsulfonhydrazide (I-24); *N*-phenyl-*N4*-(2,3-dihydro-1*H*-inden-5-oxycarbonyl)-4-methylphenylsulfonhydrazide (I-25); *N*-phenyl-*N'*-(2,3-dihydro-1*H*-inden-5-oxycarbonyl)-4-chlorophenylsulfonhydrazide (I-26); *N*-phenyl-*N*'-(2,3-dihydro-1*H*-inden-5-oxycarbonyl)-4-trifluorophenylsulfonhydrazide (I-27); and *N*-phenyl-*N'*-(2,3-dihydro-1*H*-inden-5-oxycarbonyl)-2-mesitylenesulfonhydrazide (I-28).

Further, the present invention relates to a method for preparing a *N*-phenyl-*N'-*phenoxycarbonyl-phenylsulfonhydrazide derivative of the above formula (I) comprising a step of reacting a compound of the following formula (II) with a compound of the following formula (III). 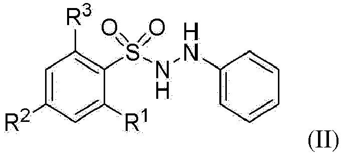 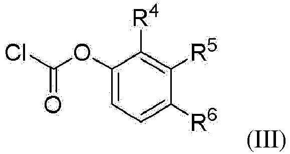 wherein, R¹, R², R³, R⁴, R⁵ and R⁶ are the same as defined in the above formula (I).

In one embodiment of the present invention, the compound of the above formula (II) is reacted with the compound of the above formula (III) in the presence of a base.

The base may be triethylamine (TEA), pyridine, *N,N*-diisopropylethylamine, etc. and preferably may be triethylamine.

The above reaction may be performed without any additional reaction solvent, or with tetrahydrofuran (THF), 1,4-dioxane, diethylether, etc as a reaction solvent. Preferably, reflux condition may be used for reaction temperature.

The compound of the above formula (II) and the compound of the above formula (III) are commercially available or may be easily prepared in accordance with a known method.

In particular, the compound of the above formula (II) may be prepared by reacting a compound of the following formula (IV) with phenyl hydrazine of the following formula (V).

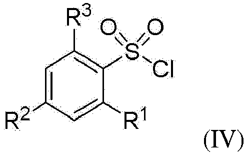

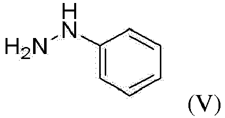

The reaction may be performed in the presence of a base and the base may be triethylamine (TEA), pyridine, *N,N*-diisopropylethylamine, etc. and preferably may be triethylamine.

The reaction solvent may be dichloromethane, chloroform, etc. and the reaction temperature is preferably room temperature.

Further, the compound of the above formula (III) may be prepared by reacting a compound of the following formula (VI) with triphosgene of the following formula (VII).

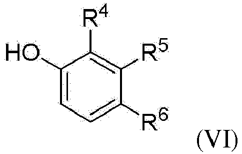

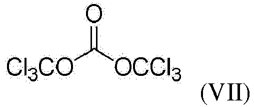

The reaction may be performed in the presence of a base and the base may be triethylamine (TEA), pyridine, *N,N*-diisopropylethylamine (DIPEA), etc. and preferably may be *N,N*-diisopropylethylamine.

The reaction solvent may be tetrahydrofuran (THF), 1,4-dioxane, diethylether, etc. and the reaction temperature is preferably 0 to 10 °C.

The compound of the above formula (I) according to the present invention or pharmaceutically acceptable salt thereof exhibits an excellent inhibitory activity on PGE₂ production by inhibiting microsomal prostaglandin E₂ synthase-1 (mPGES-1) (see Experiment Example 1).

Accordingly, the present invention relates to a pharmaceutical composition for inhibiting microsomal prostaglandin E₂ synthase-1 (mPGES-1) comprising the compound of the above formula (I) or pharmaceutically acceptable salt thereof together with a pharmaceutically acceptable carrier. In particular, the present invention relates to a pharmaceutical composition for preventing or treating inflammation, arthritis, high fever, pain, cancer, stroke or bone disease.

The pharmaceutical composition according to the present invention can be administered orally, e.g., ingestion or inhalation; or parenterally, e.g., injection, deposition, implantation or suppositories. The injection can be, for example, intravenous, subcutaneous, intramuscular or intraperitoneal. Depending on the route of administration, the pharmaceutical composition of the present invention may be formulated as tablets, capsules, granules, fine subtilae, powders, sublingual tablets, suppositories, ointments, injection solutions, emulsions, suspensions, syrups, aerosols, etc. The above various forms of the pharmaceutical composition of the present invention can be prepared in a manner well known in the art using a pharmaceutically acceptable carrier(s) which are usually used for each form. Examples of the pharmaceutically acceptable carriers include excipient, binder, disintegrating agent, lubricant, preservative, antioxidant, isotonic agent, buffer, coating agent, sweetening agent, dissolvent, base, dispersing agent, wetting agent, suspending agent, stabilizer, colorant, etc.

The pharmaceutical composition according to the present invention contains 0.01 to 95 wt% of the compound according to the present invention or pharmaceutically acceptable salt thereof depending on the form thereof.

The specific dosage of the present pharmaceutical composition can be varied with species of mammals including a human-being, body weight, gender, severity of disease, judgment of doctor, etc. It is preferable that 0.01 to 50 mg of the active ingredient is administered per kg of body weight a day for oral use, while 0.01 to 10 mg of the active ingredient is administered per kg of body weight a day for parenteral use. The total daily dosage can be administered once or over several times depending on the severity of disease, judgment of doctor, etc.

### [Advantageous Effects]

The present compound exhibits an excellent inhibitory activity on PGE₂ production by inhibiting microsomal prostaglandin E₂ synthase-1 (mPGES-1). Accordingly, the present compound may be effectively used for a pharmaceutical composition for preventing or treating inflammation, arthritis, high fever, pain, cancer, stroke or bone disease.

### [Best Mode]

The present invention is further illustrated by the following examples, which are not to be construed to limit the scope of the invention.

### Preparation Example 1: Preparation of a compound of formula II

### Preparation Example 1-1: N-phenyl-4-toluenesulfonhydrazide (II-1)

Phenyl hydrazine (2.29 g, 21.26 mmol, 1.0 eq) was dissolved in dichloromethane solvent at room temperature. To the resulting solution were added 4-toluenesulfonyl chloride (6.05 g, 31.73 mmol, 1.5 eq) and triethylamine (TEA) (4.43 ml, 31.73 mmol, 1.5 eq) at 0 °C and the resulting mixture was stirred at room temperature for 1.5 hrs. After the reaction was completed, dichloromethane solvent was removed using a rotary evaporator and the resulting residue was extracted with ethyl acetate and distilled water. Distilled water remaining in ethyl acetate was removed with anhydrous magnesium sulfate and ethyl acetate solvent was removed using a rotary evaporator. The resulting residue was recrystallized with methanol to produce a white solid which was solidified using n-hexane to give the title compound (II-1) (1.29 g, 4.90 mmol, 23%).

¹H-NMR (400 MHz, DMSO-*d*₆) δ: 9.45 (1 H, s), 7.74 (2 H, m), 7.58(1 H, s), 7.42(2 H, d, *J* = 7.6 Hz), 7.09(2 H, t, *J* = 7.2 Hz), 6.80(2 H, d, *J* = 8.0 Hz), 6.93(1 H, t, *J* = 6.8 Hz).

### Preparation Example 1-2:

### N-phenyl-4-methoxyphenylsulfonhydrazide (II-2)

The title compound (II-2) (37%) was obtained according to the same procedure as Preparation Example 1-1, except for using 4-methoxyphenylsulfonyl chloride instead of 4-toluenesulfonyl chloride.

¹H-NMR (400 MHz, DMSO-*d*₆) δ: 9.36 (1 H, s), 7.76 (2 H, d, *J* = 8.8 Hz), 7.57 (1 H, s), 7.14-7.06 (4 H, m), 6.79 (2 H, d, *J* = 8.0 Hz), 6.68 (1 H, t, *J* = 7.2 Hz), 3.84 (3 H, s).

### Preparation Example 1-3: N-phenyl-phenylsulfonhydrazide (II-3)

The title compound as a white solid (II-3) (50%) was obtained according to the same procedure as Preparation Example 1-1, except for using phenylsulfonyl chloride instead of 4-toluenesulfonyl chloride.

¹H-NMR (400 MHz, DMSO-*d*₆) δ: 9.56 (1 H, s), 7.85 (2 H, d, *J* = 7.2 Hz), 7.84-7.59 (4 H, m), 7.09 (2 H, t, *J* = 7.6 Hz), 6.81-6.71 (2 H, m), 6.69 (1 H, t, *J* = 7.2 Hz).

### Preparation Example 1-4: N-phenyl-4-chlorophenylsulfonhydrazide (II-4)

The title compound (II-4) (48%) was obtained according to the same procedure as Preparation Example 1-1, except for using 4-chlorophenylsulfonyl chloride instead of 4-toluenesulfonyl chloride.

¹H-NMR (400 MHz, DMSO-*d*₆) δ: 9.64 (1 H, s), 7.83 (2 H, d, *J* = 8.8 Hz), 7.69 (2 H, d, *J* = 8.4 Hz), 7.10 (2 H, t, *J* = 8.4 Hz), 6.78 (2 H, d, *J* = 8.4 Hz), 6.711 (1 H, t, *J* = 7.2 Hz).

### Preparation Example 1-5:

### N-phenyl-4-trifluoromethylphenylsulfonhydrazide (II-5)

The title compound (II-5) (62%) was obtained according to the same procedure as Preparation Example 1-1, except for using 4-trifluoromethylphenylsulfonyl chloride instead of 4-toluenesulfonyl chloride.

¹H-NMR (400 MHz, CDCl₃) δ: 8.01 (2 H, d, *J* = 8 Hz), 7.72 (2 H, d, *J* = 8 Hz), 7.14-7.10 (2 H, m), 6.86 (1 H, t, *J* = 7.6 Hz), 6.71 (2 H, d, *J* = 8.6 Hz), 6.42 (1 H, s).

### Preparation Example 1-6: N-phenyl-2-mesitylenesulfonhydrazide (II-6)

The title compound (II-6) (35%) was obtained according to the same procedure as Preparation Example 1-1, except for using 2-mesitylenesulfonyl chloride instead of 4-toluenesulfonyl chloride.

¹H-NMR (400 MHz, CDCl₃) δ: 7.10-7.06 (2 H, m), 6.90 (2 H, s), 6.81-6.77 (1 H, m), 6.71-6.69 (2 H, m), 6.21 (1 H, s), 2.64 (6 H, s), 2.29 (3 H, s).

### Preparation Example 1-7: N-phenyl-biphenyl-4-sulfonhydrazide (II-7)

The title compound (II-7) (42%) was obtained according to the same procedure as Preparation Example 1-1, except for using biphenyl-4-sulfonyl chloride instead of 4-toluenesulfonyl chloride.

¹H-NMR (400 MHz, CDCl₃) δ: 7.94-7.92 (2 H, m), 7.67-7.62 (2 H, m), 7.58-7.56 (2 H, m), 7.50-7.42 (3 H, m), 7.12-7.08 (2 H, m), 6.84-6.80 (1 H, m), 6.76-6.74 (2 H, m), 6.35 (1 H, s).

### Preparation Example 1-8:

### N-phenyl-4-n-propylphenylsulfonhydrazide (II-8)

The title compound (II-8) (59%) was obtained according to the same procedure as Preparation Example 1-1, except for using 4-*n*-propylphenylsulfonyl chloride instead of 4-toluenesulfonyl chloride.

¹H-NMR (400 MHz, CDCl₃) δ: 7.71 (2 H, d, *J* = 8.4 Hz), 7.17 (2 H, d, *J* = 8.4 Hz), 7.04-7.00 (2 H, m), 6.75-6.71 (1 H, m), 6.65-6.63 (2 H, m), 6.14 (1 H, s), 2.55 (2 H, t, *J* = 7.2 Hz), 1.56 (2 H, m), 0.85 (3 H, t, *J* = 7.2 Hz).

### Preparation Example 1-9: N-phenyl-4-t-butylphenylsulfonhydrazide (II-9)

The title compound (II-9) (45%) was obtained according to the same procedure as Preparation Example 1-1, except for using 4-*t*-butylphenylsulfonyl chloride instead of 4-toluenesulfonyl chloride.

¹H-NMR (400 MHz, DMSO-*d*₆) δ: 9.48 (1 H, s), 7.76 (2 H, d, *J* = 8.4 Hz), 7.64-7.62 (3 H, m), 7.08 (2 H, t, *J* = 8 Hz), 6.79 (2 H, d, *J* = 8 Hz), 6.68 (1 H, t, *J* = 7.2 Hz), 1.34 (9H, s).

### Preparation Example 1-10:

### N-phenyl-4-i-propylphenylsulfonhydrazide (II-10)

The title compound (II-10) (23%) was obtained according to the same procedure as Preparation Example 1-1, except for using 4-*i*-propylphenylsulfonyl chloride instead of 4-toluenesulfonyl chloride.

¹H-NMR (400 MHz, DMSO-*d*₆) δ: 7.81 (2 H, d, *J* = 8.4 Hz), 7.30 (2 H, t, *J* = 8.4 Hz), 7.10 (2 H, d, *J* = 7.6 Hz), 6.83 (1 H, t, *J* = 7.2 Hz), 6.73 (2 H, d, *J* = 8.4 Hz), 6.23 (1 H, s), 5.70 (3 H, s), 2.97 (1 H, m), 1.29 (6 H, d, *J* = 15.2 Hz).

### Preparation Example 2: Preparation of a compound of formula III

### Preparation Example 2-1: 4-(Benzyloxy)phenyl chloroformate (III-1)

Triphosgene (2.37 g, 7.99 mmol, 0.8 eq) was dissolved in anhydrous THF solvent at anhydrous condition. To the resulting solution was added 4-(benzyloxy)phenol (2.00 g, 9.99 mmol, 1.0 eq) dissolved in anhydrous THF solvent. To the resulting mixture was slowly added diisopropylethylamine (DIPEA) (1.74 ml, 9.99 mmol, 1.0 eq) and the resulting mixture was stirred at 0 °C for 16 hrs. And then, the mixture was extracted with ethyl acetate and distilled water, and the distilled water was removed from the resulting ethyl acetate solution using anhydrous magnesium sulfate. The ethyl acetate solvent was removed using a rotary evaporator to give the title compound as a white solid (III-1) (2.667 g, 10.15 mmol, >99 %).

¹H-NMR (400 MHz, CDCl₃) δ: 7.46-7.34 (5 H, m), 7.18-7.13 (2 H, m), 7.02-6.68 (2 H, m).

### Preparation Example 2-2: 4-(Ethoxy)phenyl chloroformate (III-2)

The title compound (III-2) (98%) was obtained according to the same procedure as Preparation Example 2-1, except for using 4-ethoxyphenol instead of 4-(benzyloxy)phenol.

¹H-NMR (400 MHz, CDCl₃) δ: 7.06-7.01 (2 H, m), 6.82-6.78 (2 H, m), 3.93 (2 H, q, *J* = 6.8 Hz), 1.33 (3 H, t, *J* = 6.8 Hz).

### Preparation Example 2-3: 4-(Phenoxy)phenyl chloroformate (III-3)

The title compound (III-3) (99%) was obtained according to the same procedure as Preparation Example 2-1, except for using 4-phenoxyphenol instead of 4-(benzyloxy)phenol.

¹H-NMR (400 MHz, CDCl₃) δ: 7.41-7.36 (2 H, m), 7.22-7.15 (3 H, m), 7.06-7.03 (4 H, m).

### Preparation Example 2-4: 4-(Ethyl)phenyl chloroformate (III-4)

The title compound (III-4) (98%) was obtained according to the same procedure as Preparation Example 2-1, except for using 4-ethylphenol instead of 4-(benzyloxy)phenol.

¹H-NMR (400 MHz, CDCl₃) δ: 7.19-7.15 (2 H, m), 7.06-7.04 (2 H, m), 2.59 (2 H, q, *J* = 7.2 Hz), 1.17 (3 H, t, *J* = 7.2 Hz).

### Preparation Example 2-5: 2,3-Dihydro-1H-inden-5-yl chloroformate (III-5)

The title compound (III-5) (98%) was obtained according to the same procedure as Preparation Example 2-1, except for using 2,3-dihydro-1H-inden-5-ol instead of 4-(benzyloxy)phenol.

¹H-NMR (400 MHz, CDCl₃) δ: 7.10 (1 H, d, *J* = 8.0 Hz), 6.93 (1H, s), 6.83 (1H, d, *J* = 8.0 Hz), 2.79 (4H. t, *J* = 7.0 Hz), 1.99 (2H, p, *J* = 7.0 Hz).

### Example 1: N-phenyl-N'-(4-methoxyphenoxycarbonyl)-4-methoxyphenylsulfonhydrazide (I-1: MPO-0047)

The compound (II-2) (0.50 g, 1.80 mmol, 1.0 eq) obtained in Preparation Example 1-2 was dissolved in a small amount of 4-methoxyphenyl chloroformate (0.34 g, 2.17 mmol, 1.2 eq). To the resulting solution was added TEA (0.180 ml, 1.29 mmol, 1.2 eq) and the resulting mixture was stirred under reflux at 50 °C for 0.5 hr. And then, the mixture was extracted with ethyl acetate and distilled water, and the distilled water was removed from the resulting ethyl acetate solution using anhydrous magnesium sulfate. The ethyl acetate solvent was removed using a rotary evaporator. And then, the resulting residue was purified using a flash column chromatography with a mixed solvent of ethyl acetate and n-hexane (1:3) and then recrystallized with diethylether and n-hexane to give the title compound as a white solid (I-1) (0.2260 g, 0.527 mmol, 49%).

¹H-NMR (400 MHz, CDCl₃) δ: 7.90-7.88 (2 H, m), 7.28-7.21 (2 H, m), 7.01-6.93 (5 H, m), 6.86-6.74 (4 H, m), 3.90 (3 H, s), 3.78 (3 H, s).

### Example 2: N-phenyl-N'-(4-methylphenoxycarbonyl)-4-methylphenylsulfonhydrazide (I-2: MPO-0048)

The title compound as a white solid (I-2) (63%) was obtained according to the same procedure as Example 1, using the compound (II-1) obtained in Preparation Example 1-1 and 4-methylphenyl chloroformate.

¹H-NMR (400 MHz, CDCl₃) δ: 7.85-7.81 (2 H, m), 7.43 (1 H, d, *J* = 8.4 Hz), 7.29-7.22 (3 H, m), 7.11 (2 H, d, *J* = 8.4 Hz), 6.98 (1 H, t, *J* = 7.6 Hz), 6.90-6.87 (2 H, m), 6.81-6.79 (2 H, m), 6.53 (1 H, s), 2.44 (3 H, s), 2.30 (3 H, s).

### Example 3: N-phenyl-N'-(2-methoxyphenoxycarbonyl)-4-methylphenylsulfonhydrazide (I-3: MPO-0049)

The title compound as a white solid (I-3) (48%) was obtained according to the same procedure as Example 1, using the compound (II-1) obtained in Preparation Example 1-1 and 2-methoxyphenyl chloroformate.

¹H-NMR (400 MHz, CDCl₃) δ: 7.83-7.81 (2 H, m), 7.28-7.22 (4 H, m), 7.20-7.16 (1 H, m), 7.01-6.95 (2 H, m), 6.91-6.87 (4 H, m), 3.65 (3 H, s), 2.43 (3 H, s).

### Example 4: N-phenyl-N'-(4-benzyloxyphenoxycarbonyl)-4-methoxyphenylsulfonhydrazide (I-4: MPO-0050)

The title compound as a white solid (I-4) (75%) was obtained according to the same procedure as Example 1, using the compound (II-2) obtained in Preparation Example 1-2 and the compound (III-1) obtained in Preparation Example 2-1.

¹H-NMR (400 MHz, DMSO-*d*₆) δ: 9.16 (1 H, s), 7.93 (2 H, d, *J* = 8.8 Hz), 7.43-7.19 (9 H, m), 7.00-6.98 (2 H, m), 6.90-6.85 (3 H, m), 6.87 (2 H, d, *J* = 8.0 Hz), 5.07 (2 H, s), 3.88 (3 H, s).

### Example 5: N-phenyl-N'-(4-ethoxyphenoxycarbonyl)-4-methoxyphenylsulfonhydrazide (I-5: MPO-0051)

The title compound as a white solid (I-5) (52%) was obtained according to the same procedure as Example 1, using the compound (II-2) obtained in Preparation Example 1-2 and the compound (III-2) obtained in Preparation Example 2-2.

¹H-NMR (400 MHz, DMSO-*d*₆) δ: 9.17 (1 H, s), 7.93 (2 H, d, *J* = 8.8 Hz), 7.27-7.19 (4 H, m), 6.91-6.83 (5 H, m), 6.78 (2 H, d, *J* = 8.0 Hz), 3.98 (2 H, q, *J* = 6.8 Hz), 1.29 (3 H, t, *J* = 6.8 Hz).

### Example 6: N-phenyl-N'-(4-phenoxyphenoxycarbonyl)-4-methoxyphenylsulfonhydrazide (I-6: MPO-0052)

The title compound as a white solid (I-6) (81%) was obtained according to the same procedure as Example 1, using the compound (II-2) obtained in Preparation Example 1-2 and the compound (III-3) obtained in Preparation Example 2-3.

¹H-NMR (400 MHz, DMSO-*d*₆) δ: 9.17 (1 H, s), 7.94 (2 H, d, *J* = 9.2 Hz), 7.39 (2 H, t, *J* = 7.2 Hz), 7.27-7.20 (4 H, m), 7.15 (1 H, t, *J* = 7.2 Hz), 7.00-6.99 (6 H, m), 6.85 (1 H, t, *J* = 7.2 Hz), 6.80 (2 H, t, *J* = 8.0 Hz), 3.88 (3 H, s).

### Example 7: N-phenyl-N'-(4-ethylphenoxycarbonyl)-4-methoxyphenylsulfonhydrazide (I-7: MPO-0053)

The title compound as a white solid (I-7) (44%) was obtained according to the same procedure as Example 1, using the compound (II-2) obtained in Preparation Example 1-2 and the compound (III-4) obtained in Preparation Example 2-4.

¹H-NMR (400 MHz, DMSO-*d*₆) δ: 9.18 (1 H, s), 7.94-7.92 (2 H, m), 7.27-7.20 (6 H, m), 6.88-6.78 (5 H, m), 3.89 (3 H, s), 2.57 (2 H, q, *J* = 7.6 Hz), 1.14 (3 H, t, *J* = 7.6 Hz).

### Example 8: N-phenyl-N'-(4-benzyloxyphenoxycarbonyl)-4-methylphenylsulfonhydrazide (I-8: MPO-0055)

The title compound as a white solid (I-8) (36%) was obtained according to the same procedure as Example 1, using the compound (II-1) obtained in Preparation Example 1-1 and the compound (III-1) obtained in Preparation Example 2-1.

¹H-NMR (400 MHz, DMSO-*d*₆) δ: 9.18 (1 H, s), 7.89 (2 H, d, *J* = 8.4 Hz), 7.58 (2 H, d, *J* = 8.4 Hz), 7.51-7.23 (7 H, m), 7.00-6.98 (2 H, m), 6.97-6.79 (5 H, m), 5.07 (2 H, s), 2.44 (3 H, s).

### Example 9: N-phenyl-N'-(2,3-dihydro-1H-inden-5-oxycarbonyl)-4-methoxyphenylsulfonhydrazide (I-9: MPO-0057)

The title compound (I-9) (10%) was obtained according to the same procedure as Example 1, using the compound (II-2) obtained in Preparation Example 1-2 and the compound (III-5) obtained in Preparation Example 2-5.

¹H NMR (400 MHz, DMSO-*d*₆) δ: 9.16 (1 H, s), 7.94-7.91 (1 H, m), 7.27-7.23 (2 H, m), 7.22-7.18 (3 H, m), 6.85 (1 H, t, *J* = 7.2 Hz), 6.81-6.77 (3 H, m), 6.72 (1 H, dd, *J₁*= 8.0 Hz, *J* = 2.4 Hz,), 3.89 (3 H, s), 2.81 (2 H, t, *J* = 7.2 Hz), 2.80 (2 H, t, *J* = 7.2 Hz), 2.00(2 H, p, *J* = 7.6 Hz).

### Example 10: N-phenyl-N'-phenoxycarbonyl-4-chlorophenylsulfonhydrazide (I-10: MPO-0058)

The title compound (I-10) (76%) was obtained according to the same procedure as Example 1, using the compound (II-4) obtained in Preparation Example 1-4 and phenyl chloroformate.

¹H-NMR (400 MHz, DMSO-*d*₆) δ: 9.24 (1 H, s), 8.02 (2 H, d, *J* = 8.8 Hz), 7.80 (2 H, d, *J* = 8.4 Hz), 7.39 (2 H, t, *J* = 8 Hz), 7.27 (3 H, t, *J* = 8 Hz), 7.00 (2 H, d, *J* = 7.6 Hz), 6.88 (1 H, q), 6.82 (2 H, d, *J* = 7.6 Hz).

### Example 11: N-phenyl-N'-(2-methoxyphenoxycarbonyl)-4-chlorophenylsulfonhydrazide (I-11: MPO-0059)

The title compound (I-11) (92%) was obtained according to the same procedure as Example 1, using the compound (II-4) obtained in Preparation Example 1-4 and 2-methoxyphenyl chloroformate.

¹H-NMR (400 MHz, DMSO-*d*₆) δ : 9.30 (1 H, s), 7.99 (2 H, d, *J* = 8.8 Hz), 7.79 (2 H, d, *J* = 8.8 Hz), 7.29-7.21 (3 H, m), 7.08 (1 H, t, *J* = 8 Hz), 7.01 (1 H, q, *J* = 8 Hz), 6.94-6.83 (4 H, m), 3.61 (3 H, s).

### Example 12: N-phenyl-N'-(4-ethylphenoxycarbonyl)-4-chlorophenylsulfonhydrazide (I-12: MPO-0060)

The title compound (I-12) (75%) was obtained according to the same procedure as Example 1, using the compound (II-4) obtained in Preparation Example 1-4 and the compound (III-4) obtained in Preparation Example 2-4.

¹H-NMR (400 MHz, DMSO-*d*₆) δ : 9.23 (1 H, s), 8.01 (2 H, d, *J* = 8.8 Hz), 7.79 (2 H, d, *J* = 8.8 Hz), 7.26 (2 H, t, *J* = 8 Hz), 7.21 (2 H, d, *J* = 8.8 Hz), 6.88 (3 H, t, *J* = 8.4 Hz), 6.80 (2 H, d, *J* = 7.6 Hz), 2.57 (2 H, q, *J* = 7.6 Hz), 1.13 (3 H, t, *J* = 7.6 Hz).

### Example 13: N-phenyl-N'-(4-ethoxyphenoxycarbonyl)-4-chlorophenylsulfonhydrazide (I-13: MPO-0061)

The title compound (I-13) (78%) was obtained according to the same procedure as Example 1, using the compound (II-4) obtained in Preparation Example 1-4 and the compound (III-2) obtained in Preparation Example 2-2.

¹H-NMR (400 MHz, DMSO-*d*₆) δ : 9.21 (1 H, s), 8.01 (2 H, d, *J* = 8.4 Hz), 7.79 (2 H, d, *J* = 8.8 Hz), 7.26 (2 H, t, *J* = 8.0 Hz), 6.87 (5 H, m), 6.81 (2 H, d, *J* = 8 Hz), 6.80 (2 H, d, *J* = 8.0 Hz), 2.57 (2 H, q), 1.29 (3 H, t, *J* = 6.8 Hz).

### Example 14: N-phenyl-N'-(4-phenoxyphenoxycarbonyl)-4-chlorophenylsulfonhydrazide (I-14: MPO-0062)

The title compound (I-14) (75%) was obtained according to the same procedure as Example 1, using the compound (II-4) obtained in Preparation Example 1-4 and the compound (III-3) obtained in Preparation Example 2-3.

¹H-NMR (400 MHz, DMSO-*d*₆) δ : 9.23 (1 H, s), 8.03-8.00 (2 H, m), 7.82-7.79 (2 H, m), 7.41-7.37 (2 H, m), 7.27 (2 H, t, *J* = 7.6 Hz), 7.15 (1 H, t, *J* = 7.6 Hz), 7.06-6.97 (6 H, m), 6.89-6.81 (3 H, m).

### Example 15: N-phenyl-N'-(4-benzyloxyphenoxycarbonyl)-4-chlorophenylsulfonhydrazide (I-15: MPO-0063)

The title compound (I-15) (54%) was obtained according to the same procedure as Example 1, using the compound (II-4) obtained in Preparation Example 1-4 and the compound (III-1) obtained in Preparation Example 2-1.

¹H-NMR (400 MHz, DMSO-*d*₆) δ: 9.22 (1 H, s), 8.11 (2 H, d, *J* = 8.8 Hz), 7.97 (2 H, d, *J* = 8.4 Hz), 7.43-7.25 (7 H, m), 7.01-6.98 (2 H, m), 6.93-6.85 (3 H, m), 6.82-6.80 (2 H, d, *J* = 8.4 Hz), 5.07 (2 H, s).

### Example 16: N-phenyl-N'-(4-phenoxyphenoxycarbonyl)-2-mesitylenesulfonhydrazide (I-16: MPO-0064)

The title compound (I-16) (56%) was obtained according to the same procedure as Example 1, using the compound (II-6) obtained in Preparation Example 1-6 and the compound (III-3) obtained in Preparation Example 2-3.

¹H-NMR (400 MHz, DMSO-*d*₆) δ: 9.03 (1 H, s), 7.38 (2 H, t, *J* = 6.8 Hz), 7.28 (2 H, t, *J* = 8.4 Hz), 7.14 (3 H, t, *J* = 7.6 Hz), 6.99-6.95 (6 H, m), 6.88-6.85 (3 H, m), 2.61 (6 H, s), 2.30 (3 H, s).

### Example 17: N-phenyl-N'-(4-benzyloxyphenoxycarbonyl)-2-mesitylenesulfonhydrazide (I-17: MPO-0065)

The title compound (I-17) (56%) was obtained according to the same procedure as Example 1, using the compound (II-6) obtained in Preparation Example 1-6 and the compound (III-1) obtained in Preparation Example 2-1.

¹H-NMR (400 MHz, DMSO-*d*₆) δ: 9.00 (1 H, s), 7.42-7.26 (7 H, m), 7.14 (2 H, s), 6.96 (2 H, d, *J* = 8.8 Hz), 6.86 (1 H, t, *J* = 7.2 Hz), 6.74 (2 H, d, *J* = 7.2 Hz), 5.05 (2 H, s), 2.60 (6 H, s), 2.30 (3 H, s).

### Example 18: N-phenyl-N'-(4-phenoxyphenoxycarbonyl)-biphenyl-4-sulfonhydrazide (I-18: MPO-0066)

The title compound (I-18) (56%) was obtained according to the same procedure as Example 1, using the compound (II-7) obtained in Preparation Example 1-7 and the compound (III-3) obtained in Preparation Example 2-3.

¹H-NMR (400 MHz, DMSO-*d*₆) δ: 9.28 (1 H, s), 8.09 (2 H, d, *J =* 8.8 Hz), 8.01 (2 H, d, *J* = 8.8 Hz), 7.79 (2 H, d, *J* = 8 Hz), 7.55 (2 H, t, *J* = 6.4 Hz), 7.52-7.46 (1 H, m), 7.37 (2 H, t, *J* = 8 Hz), 7.27 (2 H, t, *J* = 8 Hz), 7.14 (1 H, t, *J* = 7.2 Hz), 7.04-6.98 (6 H, m), 6.89-6.85 (3 H, m).

### Example 19: N-phenyl-N'-(4-benzyloxyphenoxycarbonyl)-biphenyl-4-sulfonhydrazide (I-19: MPO-0067)

The title compound (I-19) (56%) was obtained according to the same procedure as Example 1, using the compound (II-7) obtained in Preparation Example 1-7 and the compound (III-1) obtained in Preparation Example 2-1.

¹H-NMR (400 MHz, DMSO-*d*₆) δ: 9.26 (1 H, s), 8.08 (2 H, d, *J* = 6.8 Hz), 8.00 (2H, d, *J* = 6.8 Hz), 7.81-7.79 (2 H, m), 7.57-7.53 (2 H, m), 7.50-7.48 (1 H, m), 7.43-7.25 (7 H, m), 7.00-6.97 (2 H, m), 6.92-6.83 (5 H, m), 5.06 (2 H, s).

### Example 20: N-phenyl-N'-(4-phenoxyphenoxycarbonyl)-4-n-propylphenylsulfonhydrazide (I-20: MPO-0068)

The title compound (I-20) (56%) was obtained according to the same procedure as Example 1, using the compound (II-8) obtained in Preparation Example 1-8 and the compound (III-3) obtained in Preparation Example 2-3.

¹H-NMR (400 MHz, DMSO-*d*₆) δ: 9.04 (1 H, s), 7.40-7.36 (2 H, m), 7.30-7.26 (2 H, m), 7.17-7.12 (3 H, m), 7.00-6.95 (6 H, m), 6.89-6.85 (3 H, m), 2.62 (6H, s), 2.31 (3H, s).

### Example 21: N-phenyl-N'-(4-benzyloxyphenoxycarbonyl)-4-trifluoromethylphenylsulfonhydrazide (I-21: MPO-0078)

The title compound (I-21) (56%) was obtained according to the same procedure as Example 1, using the compound (II-5) obtained in Preparation Example 1-5 and the compound (III-1) obtained in Preparation Example 2-1.

¹H-NMR (400 MHz, DMSO-*d*₆) δ: 9.25 (1 H, s), 8.24 (2 H, d, *J =* 8.0 Hz), 8.11 (2H, d, *J* = 8.0 Hz), 7.43-7.31 (5 H, m), 7.30-7.25 (2 H, m), 7.00-6.97 (2 H, m), 6.94-6.82 (7 H, m), 5.07 (2 H, s), 3.51 (1 H, s).

### Example 22: N-phenyl-N-(4-benzyloxyphenoxycarbonyl)-4-t-butylphenylsulfonhydrazide (I-22: MPO-0081)

The title compound (I-22) (78%) was obtained according to the same procedure as Example 1, using the compound (II-9) obtained in Preparation Example 1-9 and the compound (III-1) obtained in Preparation Example 2-1.

¹H-NMR (400 MHz, DMSO-*d*₆) δ: 7.93 (2 H, d, *J* = 2.0 Hz), 7.73 (2 H, d, *J* = 1.6 Hz), 7.40 (4 H, m), 7.34 (1 H, t, *J* = 2.0 Hz), 7.25 (2 H, m), 6.97 (2 H, m), 6.83 (5 H, m), 5.07 (2 H, s), 1.33 (9 H, s).

### Example 23: N-phenyl-N'-(4-phenoxyphenoxycarbonyl)-4-t-butylphenylsulfonhydrazide (I-23: MPO-0084)

The title compound (I-23) (33%) was obtained according to the same procedure as Example 1, using the compound (II-9) obtained in Preparation Example 1-9 and the compound (III-3) obtained in Preparation Example 2-3.

¹H-NMR (400 MHz, DMSO-*d*₆) δ: 9.21 (1 H, s), 7.95 (2 H, d, *J* = 8.4 Hz), 7.73 (2 H, d, *J* = 8.4 Hz), 7.39 (2 H, t, *J* = 7.6 Hz), 7.26 (2 H, t, *J* = 7.6 Hz), 7.15 (1 H, t, *J* = 7.2 Hz), 6.98 (6 H, m), 6.83 (3 H, m), 1.33 (9 H, s).

### Example 24: N-phenyl-N'-(4-phenoxyphenoxycarbonyl)-4-i-propylphenylsulfonhydrazide (I-24: MPO-0085)

The title compound (I-24) (45%) was obtained according to the same procedure as Example 1, using the compound (II-10) obtained in Preparation Example 1-10 and the compound (III-3) obtained in Preparation Example 2-3.

¹H-NMR (400 MHz, DMSO-*d*₆) δ: 9.20 (1 H, s), 7.94 (2 H, d, *J* = 8.4 Hz), 7.58 (2 H, d, *J* = 8.4 Hz), 7.40 (2 H, d, *J* = 2.4 Hz), 7.38 (2 H, t, *J* = 7.6 Hz), 7.25 (1 H, t, *J* = 7.2 Hz), 6.98 (6 H, m), 6.83 (3 H, m), 3.30 (1 H, m) 1.24 (6 H, d, *J* = 6.8 Hz).

### Example 25: N-phenyl-N'-(2,3-dihydro-1H-inden-5-oxycarbonyl)-4-methylphenylsulfonhydrazide (I-25: MPO-0086)

The title compound (I-25) (43%) was obtained according to the same procedure as Example 1, using the compound (II-1) obtained in Preparation Example 1-1 and the compound (III-5) obtained in Preparation Example 2-5.

¹H-NMR (400 MHz, DMSO-*d*₆) δ: 9.19 (1 H, s), 7.88 (2 H, d, *J* = 8.4 Hz), 7.50 (2H, d, *J* = 8.4 Hz), 7.25 (2 H, t, *J* = 8.0 Hz), 7.18 (1 H, d, *J* = 8.4 Hz), 6.85 (1 H, t, *J* = 7.2 Hz), 6.79 (3 H, d, *J* = 7.6 Hz), 6.65 (1 H, dd, *J* = 8.0 and 2.4 Hz), 2.80 (4 H, t, *J* = 7.6 Hz), 2.50 (3 H, m), 1.99 (2 H, p. *J* = 2 Hz).

### Example 26: N-phenyl-N'-(2,3-dihydro-1H-inden-5-oxycarbonyl)-4-chlorophenylsulfonhydrazide (I-26: MPO-0087)

The title compound (I-26) (57%) was obtained according to the same procedure as Example 1, using the compound (II-4) obtained in Preparation Example 1-4 and the compound (III-5) obtained in Preparation Example 2-5.

¹H-NMR (400 MHz, DMSO-*d*₆) δ: 9.23 (1 H, s), 8.01 (2 H, dt, *J* = 9.2 and 2.8 Hz), 7.80 (2 H, dt, *J* = 9.2 and 2.4 Hz), 7.26 (2 H, t, *J* = 8 Hz), 7.19 (1 H, d, *J* = 8 Hz), 6.88-6.80 (4 H, m), 6.69 (1 H, dd, *J* = 8.0 and 2.4 Hz), 2.83-2.78 (4H, m), 2.04-1.63 (2H, m).

### Example 27: N-phenyl-N'-(2,3-dihydro-1H-inden-5-oxycarbonyl)-4-trifluorophenylsulfonhydrazide (I-27: MPO-0088)

The title compound (I-27) (23%) was obtained according to the same procedure as Example 1, using the compound (II-5) obtained in Preparation Example 1-5 and the compound (III-5) obtained in Preparation Example 2-5.

¹H-NMR (400 MHz, DMSO-*d*₆) δ: 9.26 (1 H, s), 8.23 (2 H, d, *J* = 8.4 Hz), 8.12 (2 H, d, *J* = 8.4 Hz), 7.27 (2 H, t, *J* = 7.8 Hz), 7.19 (1 H, d, *J* = 8.4 Hz), 6.89-6.81 (4 H, m), 6.70 (1 H, dd, *J* = 8, 2 Hz), 2.80 (4 H, t, *J* = 7.6 Hz), 1.99 (2 H, p, *J* = 7.6 Hz).

### Example 28: N-phenyl-N'-(2,3-dihydro-1H-inden-5-oxycarbonyl)-2-mesitylenesulfonhydrazide (I-28: MPO-0089)

The title compound (I-28) (48%) was obtained according to the same procedure as Example 1, using the compound (II-6) obtained in Preparation Example 1-6 and the compound (III-5) obtained in Preparation Example 2-5.

¹H-NMR (400 MHz, DMSO-*d*₆) δ: 9.01 (1 H, s), 7.28 (2 H, t, *J* = 7.6 Hz), 7.14 (3 H, s), 6.96 (2 H, d, *J* = 7.6 Hz), 6.86 (1 H, t, *J* = 7.2 Hz), 6.45 (1 H, s), 6.52 (1 H, d, *J* = 7.2 Hz), 2.78 (4 H, t, *J* = 6.8 Hz), 2.60 (6 H, s), 2.30 (3 H, s), 1.98 (2 H, p, *J* = 6.8 Hz).

### Comparative Example 1: N-phenyl-N'-(phenoxycarbonyl)-phenylsulfonhydrazide (1: MPO-0046)

The compound (II-3) (0.450 g, 1.81 mmol, 1.0 eq) obtained in Preparation Example 1-3 was dissolved in phenyl chloroformate. To the resulting solution was added TEA (0.455 ml, 3.27 mmol, 1.8 eq) and the resulting mixture was stirred under reflux at 50 °C for 0.5 hr. And then, the mixture was extracted with ethyl acetate and distilled water, and the distilled water was removed from the resulting ethyl acetate solvent using anhydrous magnesium sulfate. The ethyl acetate solvent was removed using a rotary evaporator. And then, the resulting residue was recrystallized with methanol and solidified using n-hexane to give the title compound (1) as a white solid (0.560 g, 1.52 mmol, 82%).

¹H-NMR (400 MHz, DMSO-*d₆*) δ: 9.25 (1 H, s), 8.02 (2 H, t, *J* = 8 Hz), 7.82 (1 H, t, *J* = 7.6 Hz), 7.70 (2 H, t, *J* = 8.4 Hz), 7.38 (2 H, t, *J* = 6.4 Hz), 7.26 (3 H, t, *J* = 8 Hz), 6.94 (2 H, d, *J* = 7.6 Hz), 6.84 (3 H, m)

### Experiment Example 1: Inhibitory activity on PGE2 production

Raw264.7 cells (macrophage cell line from mouse) were obtained from Korean Cell Line Bank (KCLB) and cultured in Dulbecco's modified Eagle's medium (DMEM) containing 10% Fetal Bovine Serum (FBS), penicillin (100 units/mL), streptomycin sulfate (100*µ*g/mL) at 37 °C and 5% CO₂ in humidified environment. RAW264.7 cells at 5×10⁵ cells/mL were seeded at 1 mL/well in 24 wells using DMEM, left overnight, replaced with a fresh medium and then treated with a test compound of an appropriate concentration. The cells were incubated for 1 hr and then treated with LPS at 1*µ*g/mL and incubated for 24 hrs (or proper period). A supernatant was taken and diluted five times. 150 µL of an analysis buffer was added to Non Specific Binding (NSB) well, and 100 µL of an analysis buffer was added to zero point standard (B0) well. 100 µL of a standard sample was added to the other wells and 50 µL of PGE₂ conjugate was added (excluding NSB). 50 µL of PGE₂ antibody solution was added and wells were shaken for 2 hrs. Each well was suctioned and washed with a wash buffer five times. Each 200 µL of para-Nitrophenyl phosphate (pNPP) substrate was added to the all wells and the wells were stored in a bench at room temperature for 1 hr. 50 µL of stop solution was added and an absorbance was measured at 405 nm. The amount of PGE₂ was quantified using the measured absorbance and a standard curve and compared to a group treated with LPS only to obtain a concentration inhibiting 50% (IC₅₀). The results are shown in the following Table 1 and the presented values are means of three measurements.

**[Table 1]**

| | Substituent | | | | | | Cell viability (µM) | Inhibition of PGE₂ production | |
|---|---|---|---|---|---|---|---|---|---|
| | R¹ | R² | R³ | R⁴ | R⁵ | R⁶ | | % (µM)^{a} | IC₅₀ (nM) |
| Example 1 | H | MeO | H | H | H | MeO | >10 | 85.6(1) | 490 |
| Example 2 | H | Me | H | H | H | Me | >10 | 49.7 (1) | 1010 |
| Example 3 | H | Me | H | MeO | H | H | >0.1 | 91.9(0.1) | 34.05 |
| Example 4 | H | MeO | H | H | H | BnO | >1 | 62.8 (0.1) | 50.00 |
| Example 5 | H | MeO | H | H | H | EtO | >0.1 | 94.1 (0.1) | 20.52 |
| Example 6 | H | MeO | H | H | H | PhO | >0.1 | 93.4 (0.1) | 17.06 |
| Example 7 | H | MeO | H | H | H | Et | >0.1 | 90.9 (0.1) | 28.49 |
| Example 8 | H | Me | H | H | H | BnO | >0.1 | 66.6 (0.1) | 32.62 |
| Example 9 | H | MeO | H | H | CH₂CH₂CH₂ | | >0.1 | 79.5 (0.01) | 4.50 |
| Example 10 | H | Cl | H | H | H | H | >10 | 81.8 (0.01) | 5.42 |
| Example 11 | H | Cl | H | MeO | H | H | >10 | 85.9 (0.01) | 3.52 |
| Example 12 | H | Cl | H | H | H | Et | >10 | 80.5 (0.01) | 3.70 |
| Example 13 | H | Cl | H | H | H | EtO | >10 | 74.8 (0.01) | 5.02 |
| Example 14 | H | Cl | H | H | H | PhO | >10 | 67.8 (0.01) | 5.09 |
| Example 15 | H | Cl | H | H | H | BnO | >10 | 84.5 (0.01) | 3.42 |
| Example 16 | Me | Me | Me | H | H | PhO | >1 | 39.8 (0.1) | 176.92 |
| Example 17 | Me | Me | Me | H | H | BnO | >1 | 60.0 (0.1) | 81.51 |
| Example 18 | H | Ph | H | H | H | PhO | >1 | 39.3 (0.1) | 160.71 |
| Example 19 | H | Ph | H | H | H | BnO | >1 | 68.1 (0.1) | 93.04 |
| Example 20 | H | *n*-Pr | H | H | H | PhO | >10 | 45.4 (0.1) | 133.83 |
| Example 21 | H | CF₃ | H | H | H | BnO | >0.1 | 65.5 (0.1) | 47.10 |
| Example 22 | H | *t*-Bu | H | H | H | BnO | >0.1 | 69.8 (0.1) | 56.89 |
| Example 23 | H | *t*-Bu | H | H | H | PhO | >1 | 67.9 (0.1) | 99.60 |
| Example 24 | H | *i*-Pr | H | H | H | PhO | >1 | 65.4 (0.1) | 130.30 |
| Example 25 | H | Me | H | H | CH₂CH₂CH₂ | | >1 | 40.4 (0.1) | 66.72 |
| Example 26 | H | Cl | H | H | CH₂CH₂CH₂ | | >1 | 45.8 (0.1) | 70.46 |
| Example 27 | H | CF₃ | H | H | CH₂CH₂CH₂ | | >1 | 51.1 (0.1) | 55.77 |
| Example 28 | Me | Me | Me | H | CH₂CH₂CH₂ | | >1 | 57.2 (0.1) | 58.58 |
| Comparativ e Example 1 | H | H | H | H | H | H | >10 | 84.4 (10) | 5700 |

| | | | | | | | | | |
|---|---|---|---|---|---|---|---|---|---|
| ^{a} Inhibition of PGE₂ production (%) when treated with the concentration in parentheses | | | | | | | | | |

As shown in Table 1, N-phenyl-N'-phenoxycarbonyl-phenylsulfonhydrazide compounds according to the present invention have IC₅₀ of 3.42 to 1010 nM showing an excellent inhibitory activity on PGE₂ production compared to the compound of Comparative Example 1 with IC₅₀ of 5,700 nM.

### Experiment Example 2: Measurement of cell viability

Raw264.7 cells (macrophage cell line from mouse) were obtained from Korean Cell Line Bank (KCLB) and cultured in Dulbecco's modified Eagle's medium containing 10% FBS, penicillin (100 units/ml), streptomycin sulfate (100*µ*g/ml) at 37 °C and 5% CO₂ in humidified environment.

Cells were collected by centrifugation and scraping, and seeded at 1×10⁵ cells/well in a 96 well plate containing 100*µ*l of RPMI 1640 medium with 10% FBS. 3β, 4β-epoxy-8α-isobutyryloxyguaia-1(10), 11, (13)-diene-12.6α-olide was dissolved in dimethylsulfoxide (DMSO) solvent and the concentration of DMSO was not more than 0.1% in all experiments. Next day, test compounds and LPS (1*µ*g/mL) were added and the plate were cultured for 24 hrs. After washing cells once, 50*µ*ℓ of the medium containing 5mg/mℓ MTT but without FBS was added and cells were cultured at 37 °C for 4 hrs. And then, the medium was removed, formazan blue formed in the cells was dissolved in 100*µℓ* of DMSO, and an absorbance was measured at 540nm to obtain IC₅₀ values for cell toxicity. IC₅₀ means a concentration exhibiting 50% reduction of cell number compared to no treatment with a compound. The results are shown in the above Table 1 and the values are means of three measurements.

## Claims

1. A *N*-phenyl-*N'*-phenoxycarbonyl-phenylsulfonhydrazide derivative of the following formula (I) or pharmaceutically acceptable salt thereof: 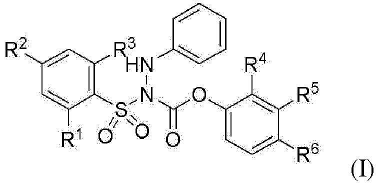 wherein,
R¹ is hydrogen, C₁-C₆ alkyl or C₁-C₆ alkoxy,
R² is C₁-C₆ alkyl, C₁-C₆ alkoxy, halogen, C₁-C₆ haloalkyl or aryl,
R³ is hydrogen, C₁-C₆ alkyl or C₁-C₆ alkoxy,
R⁴ is hydrogen, C₁-C₆ alkyl or C₁-C₆ alkoxy,
R⁵ is hydrogen, C₁-C₆ alkyl or C₁-C₆ alkoxy, and
R⁶ is hydrogen, C₁-C₆ alkyl, C₁-C₆ alkoxy or aryloxy, or
R⁵ and R⁶ are taken together with the carbon atom to which they are attached to form a 4 to 7-membered hydrocarbon ring.

2. The *N*-phenyl-*N*'-phenoxycarbonyl-phenylsulfonhydrazide derivative according to claim 1 or pharmaceutically acceptable salt thereof,
wherein,
R¹ is hydrogen or C₁-C₆ alkyl,
R² is C₁-C₆ alkyl, C₁-C₆ alkoxy, halogen, C₁-C₆ haloalkyl or aryl,
R³ is hydrogen or C₁-C₆ alkyl,
R⁴ is hydrogen or C₁-C₆ alkoxy,
R⁵ is hydrogen, and
R⁶ is hydrogen, C₁-C₆ alkyl, C₁-C₆ alkoxy or aryloxy, or
R⁵ and R⁶ are taken together with the carbon atom to which they are attached to form a 4 to 7-membered hydrocarbon ring.

3. The *N*-phenyl-*N*'-phenoxycarbonyl-phenylsulfonhydrazide derivative according to claim 1 or pharmaceutically acceptable salt thereof,
wherein,
R¹ is hydrogen or methyl,
R² is methyl, n-propyl, i-propyl, t-butyl, methoxy, chloro, trifluoromethyl or phenyl,
R³ is hydrogen or methyl,
R⁴ is hydrogen or methoxy,
R⁵ is hydrogen, and
R⁶ is hydrogen, methyl, ethyl, methoxy, ethoxy, phenoxy or benzyloxy, or
R⁵ and R⁶ are taken together with the carbon atom to which they are attached to form a 5-membered hydrocarbon ring.

4. The *N*-phenyl-*N*'-phenoxycarbonyl-phenylsulfonhydrazide derivative according to claim 1 or pharmaceutically acceptable salt thereof selected from the group consisting of the following compounds:
*N*-phenyl-*N*'-(4-methoxyphenoxycarbonyl)-4-methoxyphenylsulfonhydrazide (I-1);
*N*-phenyl-*N*'-(4-methylphenoxycarbonyl)-4-methylphenylsulfonhydrazide (I-2);
*N*-phenyl-*N*'-(2-methoxyphenoxycarbonyl)-4-methylphenylsulfonhydrazide (I-3);
*N*-phenyl-*N*'-(4-benzyloxyphenoxycarbonyl)-4-methoxyphenylsulfonhydrazide (I-4);
*N*-phenyl-*N*'-(4-ethoxyphenoxycarbonyl)-4-methoxyphenylsulfonhydrazide (I-5);
*N*-phenyl-*N*'-(4-phenoxyphenoxycarbonyl)-4-methoxyphenylsulfonhydrazide (I-6);
*N*-phenyl-*N*'-(4-ethylphenoxycarbonyl)-4-methoxyphenylsulfonhydrazide (I-7);
*N*-phenyl-*N*'-(4-benzyloxyphenoxycarbonyl)-4-methylphenylsulfonhydrazide (I-8);
*N*-phenyl-*N*'-(2,3-dihydro-1*H*-inden-5-oxycarbonyl)-4-methoxyphenylsulfonhydrazide (I-9);
*N*-phenyl-*N*'-phenoxycarbonyl-4-chlorophenylsulfonhydrazide (1-10);
*N*-phenyl-*N*'-(2-methoxyphenoxycarbonyl)-4-chlorophenylsulfonhydrazide (1-11);
*N*-phenyl-*N*'-(4-ethylphenoxycarbonyl)-4-chlorophenylsulfonhydrazide (I-12);
*N*-phenyl-*N*'-(4-ethoxyphenoxycarbonyl)-4-chlorophenylsulfonhydrazide (I-13);
*N*-phenyl-*N*'-(4-phenoxyphenoxycarbonyl)-4-chlorophenylsulfonhydrazide (I-14);
*N*-phenyl-*N*'-(4-benzyloxyphenoxycarbonyl)-4-chlorophenylsulfonhydrazide (I-15);
*N*-phenyl-*N*'-(4-phenoxyphenoxycarbonyl)-2-mesitylenesulfonhydrazide (I-16);
*N*-phenyl-*N*'-(4-benzyloxyphenoxycarbonyl)-2-mesitylenesulfonhydrazide (I-17);
*N*-phenyl-*N*'-(4-phenoxyphenoxycarbonyl)-biphenyl-4-sulfonhydrazide (I-18);
*N*-phenyl-*N*'-(4-benzyloxyphenoxycarbonyl)-biphenyl-4-sulfonhydrazide (I-19);
*N*-phenyl-*N*'-(4-phenoxyphenoxycarbonyl)-4-*n*-propylphenylsulfonhydrazide (I-20);
*N*-phenyl-*N*'-(4-benzyloxyphenoxycarbonyl)-4-trifluoromethylphenylsulfonhydrazide (I-21);
*N*-phenyl-*N*'-(4-benzyloxyphenoxycarbonyl)-4-*t*-butylphenylsulfonhydrazide (I-22);
*N*-phenyl-*N*'-(4-phenoxyphenoxycarbonyl)-4-*t*-butylphenylsulfonhydrazide (I-23);
*N*-phenyl-*N*'-(4-phenoxyphenoxycarbonyl)-4-*i*-propylphenylsulfonhydrazide (I-24);
*N*-phenyl-*N*'-(2,3-dihydro-1*H*-inden-5-oxycarbonyl)-4-methylphenylsulfonhydrazide (I-25);
*N*-phenyl-*N*'-(2,3-dihydro-1*H*-inden-5-oxycarbonyl)-4-chlorophenylsulfonhydrazide (I-26);
*N*-phenyl-*N*'-(2,3-dihydro-1*H*-inden-5-oxycarbonyl)-4-trifluorophenylsulfonhydrazide (I-27); and
*N*-phenyl-*N'*-(2,3-dihydro-1*H*-inden-5-oxycarbonyl)-2-mesitylenesulfonhydrazide (I-28).

5. A method for preparing a *N*-phenyl-*N'*-phenoxycarbonyl-phenylsulfonhydrazide derivative of the following formula (I) comprising a step of reacting a compound of the following formula (II) with a compound of the following formula (III): 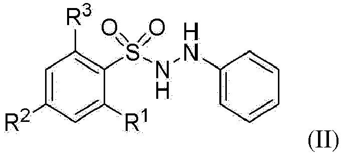 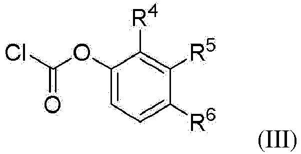 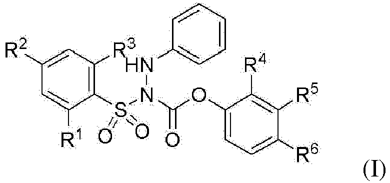 wherein,
R¹ is hydrogen, C₁-C₆ alkyl or C₁-C₆ alkoxy,
R² is C₁-C₆ alkyl, C₁-C₆ alkoxy, halogen, C₁-C₆ haloalkyl or aryl,
R³ is hydrogen, C₁-C₆ alkyl or C₁-C₆ alkoxy,
R⁴ is hydrogen, C₁-C₆ alkyl or C₁-C₆ alkoxy,
R⁵ is hydrogen, C₁-C₆ alkyl or C₁-C₆ alkoxy, and
R⁶ is hydrogen, C₁-C₆ alkyl, C₁-C₆ alkoxy or aryloxy, or
R⁵ and R⁶ are taken together with the carbon atom to which they are attached to form a 4 to 7-membered hydrocarbon ring.

6. The method according to claim 5, wherein the compound of formula (II) is reacted with the compound of formula (III) in the presence of a base.

7. The method according to claim 6, wherein the base is triethylamine.

8. The method according to claim 5, wherein the reaction is performed without any additional reaction solvent.

9. A pharmaceutical composition for inhibiting microsomal prostaglandin E₂ synthase-1 (mPGES-1) comprising the N-phenyl-N'-phenoxycarbonyl-phenylsulfonhydrazide derivative according to any one of claims 1 to 4 or pharmaceutically acceptable salt thereof together with a pharmaceutically acceptable carrier.

10. The pharmaceutical composition according to claim 9 for preventing or treating inflammation, arthritis, high fever, pain, cancer, stroke or bone disease.
